# EUROPEAN PATENT SPECIFICATION

(11) **EP 1 959 900 B1**
(45) Date of publication and mention of the grant of the patent: **29.02.2012**
(21) Application number: 05818883.0
(22) Date of filing: 16.12.2005
(51) Int. Cl.: A61F 13/42, G01V 15/00, A61B 5/00

(54) **METHOD AND SYSTEM FOR ASSOCIATING A READING OF A MACHINE READABLE SENSOR POSITIONED IN AN ABSORBENT ARTICLE WITH THE IDENTITY OF A USER**
VERFAHREN UND SYSTEM ZUR ASSOZIATION EINES MESSWERTS EINES MASCHINELL LESBAREN SENSORS IN EINEM SAUGFÄHIGEN ARTIKEL MIT DER IDENTITÄT EINES ANWENDERS
PROCÉDÉ ET SYSTÈME POUR ASSOCIER UNE LECTURE D'UN CAPTEUR LISIBLE PAR UNE MACHINE POSITIONNÉ DANS UN ARTICLE ABSORBANT À L'IDENTITÉ D'UN UTILISATEUR

(43) Date of publication of application: 27.08.2008
(73) Proprietor: SCA Hygiene Products AB, 405 03 Göteborg (SE)
(72) Inventor: TORSTENSSON, Robert, S-SE-414 59 Göteborg (SE); SAHLIN, Christian, S-SE-412 59 Göteborg (SE)
(74) Representative: VALEA AB
(86) International application number: PCT/SE2005/001971
(87) International publication number: WO 2007/069968

(56) References cited:
- EP-A1- 0 911 000
- WO-A1-2004/021944
- WO-A1-2004/021944
- WO-A2-2005/017683
- WO-A2-2005/017683
- US-A1- 2002 145 525
- US-A1- 2002 145 525
- US-A1- 2004 220 538
- US-A1- 2005 046 578

## Description

### TECHNICAL FIELD

The present invention relates to a method for associating a reading of a machine readable sensor positioned in an absorbent article, such as a diaper, a diaper of pant type, an incontinence garment, a sanitary napkin, a tampon-like product, a wound or sore dressing, a bed protector, or a similar product, with the identity of a user of the absorbent article, whereby the absorbent article is arranged to absorb or receive body discharges from the user, especially body fluid discharged from the user. Furthermore, the present invention relates to a system comprising an absorbent article, such as a diaper, a diaper of pant type, an incontinence garment, a sanitary napkin, a tampon-like product, a wound or sore dressing, a bed protector, or a similar product, and a machine readable identity tag for determination of the identity of a user of the absorbent article, whereby the absorbent article is arranged to be positioned to absorb or receive body discharges from the user, especially body fluid discharged from the user, and whereby a machine readable sensor is positioned in the absorbent article.

### BACKGROUND OF THE INVENTION

There are many different types of absorbent articles, such as diapers, diapers of pant type, incontinence garments, sanitary napkins, tampon-like products, wound or sore dressings and bed protectors, known today for absorption, retention and isolation of body wastes, such as urine, faeces and blood. Some of the known such absorbent articles comprise a sensor for detection of an event, such as urination or defecation, after absorption onto or into the absorbent article. Such a sensor may, for example, be based on detection of wetness, a biological analyte and/or a chemical analyte. When such a sensor detects an event, such as urination or defecation, a detectable response is generated. By means of the response, the user, parent, care taker, nursing personnel, etc. may determine with ease that an event has occurred. Thus, sensors in absorbent articles for detection of an event may be utilized to easily monitor if an event has occurred, i.e. to easily register the body waste status in an absorbent article.

There are many different types of sensors known today that may be utilized in an absorbent article for detection of an event One type of sensor that is utilized in some absorbent articles is the magnetoelastic sensor. Magnetoelastic sensors have been described by Grimes et al. (Biomedical Microdevices, 2:51-60, 1999).

A magnetoelastic sensor comprises a piece, typically a strip, of a magnetoelastic material. When excited by an external magnetic field, a magnetoelastic material stores magnetic energy in a magnetoelastic mode. When the magnetic field is switched off, the magnetoelastic material shows damped oscillation with a specific frequency denoted as the magnetoacoustic resonant frequency. These oscillations give rise to a magnetic flux that varies in time, which can be remotely detected by a pick-up coil. Changes in the magnetoacoustic resonant frequency may be monitored so as to measure or detect multiple environmental parameters.

An absorbent article comprising a magnetoelastic sensor is, for example, described in WO 2004/021944. More specifically, WO 2004/021944 describes a disposable sensoring absorbent structure comprising at least one absorbent layer and at least one sensing device comprising a magnetoelastic film. The sensoring absorbent structure may be comprised in an absorbent article such as, for example, a diaper, a diaper of pant type, an incontinence protector, a sanitary napkin or a bed protector. In one embodiment the sensing device is intended to be utilized for detection of wetness. The magnetoelastic film of the sensing device is then coated with a wetness sensitive polymer which interacts with wetness, e.g. moisture, liquid or humidity. The wetness sensitive polymer may interact with wetness, such as urine, through absorption or adsorption, whereby the mass of the sensing device is changed. This change in mass will either increase or decrease the magnetoacoustic resonant frequency. Thus, the mass change is measurable and correlates to the amount of wetness interacting with the wetness sensitive polymer. In another embodiment, the magnetoelastic film of the sensing device is coated directly or indirectly with at least one detector molecule adapted to detect at least one target biological and/or chemical analyte in body waste, body exudates or the users/wearers skin.

It is also known to utilize a radiofrequency (RF) tag including an inductor-capacitor resonator as a sensor for detection of an event in an absorbent article. For example, this is described in US 6,774,800. The RF tag including an inductor-capacitor resonator described in US 6,774,800 may be utilized in an energy absorption mode or an energy radiation mode. In the energy absorption mode, the RF tag selectively absorbs energy from an excitation signal at its resonant frequency. This absorption produces a unique change in the excitation signal that may be detected. However, when discharged fluid contacts the inductor-capacitor resonator of the RF tag, electrolytes in the discharged fluid create low resistance paths which detune the RF tag and thereby change its resonant frequency. Thereby, a change of the resonant frequency may be utilized to detect discharged fluid. Furthermore, in the energy radiation mode the RF tag commences oscillating at its resonant frequency in response to receiving an excitation signal. After termination of the excitation signal, energy stored in the RF tag causes the RF tag to continue oscillating at its resonant frequency. A response signal is thereby generated. In the presence of discharged fluid, the resonant frequency of the response signal is changed. Thereby, a change of the resonant frequency of the response signal may be utilized to detect discharged fluid.

In addition, it is also known to utilize a radio frequency identification (RFID) tag as a sensor for detection of an event in an absorbent article. For example, this is also described in US 6,774,800. The RFID tag utilized in US 6,774,800 may be any one of the RFID tags known in the art of electronic article surveillance (EAS) that operate in the energy absorption mode or in the energy radiation mode. An RFID tag that operates in the energy absorption mode absorbs energy from an excitation signal in one or more unique frequencies or unique bands of frequencies. However, when discharged fluid is present, the fluid attenuates the excitation signal received by such an RFID tag whereby the selective absorption of energy by the RFID tag is reduced. Thereby, discharged fluid may be detected. Furthermore, an RFID tag that operates in the energy radiation mode outputs a unique response signal including one or more frequencies or bands of frequencies in response to receiving an excitation signal. However, when discharged fluid is present, the fluid attenuates the excitation signal received by such an RFID tag and/or the response signal of the RFID tag whereby an amplitude of one or more frequencies or bands of frequencies of the response signal is reduced. Thereby, discharged fluid may be detected.

Furthermore, US 6,774,800 describes also that in addition to the use of RFID tags as sensors for detection of an event in absorbent articles, RFID tags that produce patterns of unique frequencies or bands of frequencies in either the energy absorption mode or energy radiation mode of operation can also be utilized for user identification, e.g. patient identification. Specifically, some prior art RFID tags can absorb energy from plural, e.g. up to 64, unique frequencies or bands of frequencies of an excitation signal or can output a response signal having plural, e.g. up to 64, unique frequencies or bands of frequencies. In use for patient identification such RFID tags are affixed to absorbent articles. Each user, e.g. each patient, in a particular location, e.g. a nursing home, is then associated with a pattern of frequencies or bands of frequencies produced by RFID tags that is unique from patterns of frequencies or bands of frequencies associated with other patients. Each user may then only use absorbent articles having RFID tags including the unique pattern of frequencies or bands of frequencies assigned to that user.

However, when using absorbent articles having RFID tags including a unique pattern of frequencies or band of frequencies according to US 6,774,800 there is a risk that a specific user is provided with an incorrect absorbent article, i.e. that a specific user is provided with an absorbent article having an RFID tag including a unique pattern of frequencies or band of frequencies that is assigned to another user. Thus, there is a mix-up risk, which may lead to erroneous data in journals, case books or the like and thereby to an erroneous decision of when an absorbent article has to be exchanged, etc.

### SUMMARY OF THE INVENTION

One object of the present invention is to provide an improved method for associating a reading of a machine readable sensor positioned in an absorbent article, such as a diaper, a diaper of pant type, an incontinence garment, a sanitary napkin, a tampon-like product, a wound or sore dressing, a bed protector, or a similar product, with the identity of a user of the absorbent article, whereby the absorbent article is arranged to absorb or receive body discharges from the user, by which method the reading of a machine readable sensor positioned in an absorbent article in a secure way may be associated with the identity of the user such that the above mentioned mix-up risk is eliminated or at least reduced.

This object is achieved in accordance with the characterizing portion of claim 1.

Another object of the present invention is to provide an improved system comprising an absorbent article, such as a diaper, a diaper of pant type, an incontinence garment, a sanitary napkin, a tampon-like product, a wound or sore dressing, a bed protector, or a similar product, and a machine readable identity tag for determination of the identity of a user of the absorbent article, whereby the absorbent article is arranged to be positioned to absorb or receive body discharges from the user and whereby a machine readable sensor is positioned in the absorbent article, by which system the reading of a machine readable sensor positioned in an absorbent article in a secure way may be associated with the identity of the user such that the above mentioned mix-up risk is eliminated or at least reduced.

This object is achieved in accordance with the characterizing portion of claim 13.

Still other objects and features of the present invention will become apparent from the following detailed description considered in conjunction with the accompanying drawings. It is to be understood, however, that the drawing is designed solely for purposes of illustration and not as a definition of the limits of the invention, for which reference should be made to the appended claims. It should be further understood that the drawing is not necessarily drawn to scale and that, unless otherwise indicated, it is merely intended to conceptually illustrate the structures described herein.

### BRIEF DESCRIPTION OF THE DRAWING

In the drawing:

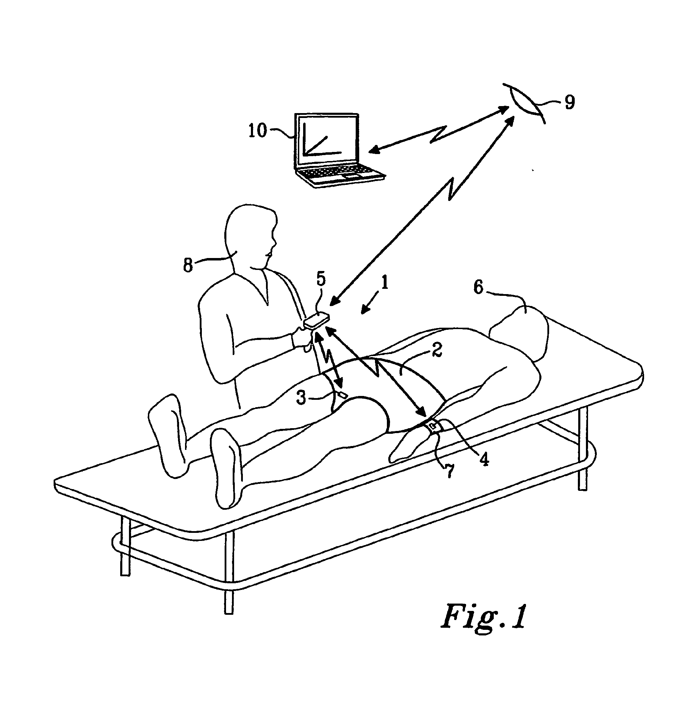

Figure 1 shows a schematic view of one embodiment of a system according to the invention and its use.

### DETAILED DESCRIPTION OF PREFERRED EMBODIMENTS

The present invention provides a method and a system, by which a reading of a machine readable sensor positioned in an absorbent article may be associated with the identity of a user of the absorbent article. The method and the system according to the invention will now be described with reference to figure 1, in which a schematic view of one embodiment of a system 1 according to the invention and its use are shown. The system 1 according to the invention comprises an absorbent article 2 in which a machine readable sensor 3 is positioned. Furthermore, the system 1 comprises a machine readable identity tag 4 for user identity determination and reading means 5.

The absorbent article 2 comprised in the system 1 according to the invention is arranged to be positioned to absorb or receive body discharges, such as discharged body fluids, body waste or body exudates, i.e. urine, faeces, blood, menstruation blood, fluid matters from wounds and sores, rinsing fluid or saliva, from a user 6, e.g. a patient, an infant, an elderly incontinent person, etc. In figure 1, the absorbent article 2 is a diaper of pant type comprising a machine readable sensor 3. However, in other embodiments the absorbent article 2 may be any other absorbent article which comprises a machine readable sensor 3 and of which the main purpose normally is to absorb, retain and isolate body discharges. For example, the absorbent article 2 may in other embodiments be a diaper, an incontinence garment, a sanitary napkin, a tampon-like product, a wound or sore dressing, a bed protector or a similar product comprising a machine readable sensor 3. The absorbent article 2 may, for example, be any of the absorbent articles described in WO 2004/021944.

The machine readable sensor 3 positioned in the absorbent article 2 may be any prior art machine readable sensor that may detect wetness, e.g. liquid, humidity or moisture, a biological analyte and/or a chemical analyte in an absorbent article 2 and that is realized as a wireless communication means. Examples of biological analytes and chemical analytes will be given below. The sensor 3 may be positioned in contact with or in spaced relation with an absorbent material of an absorbent structure of the absorbent article 2. Furthermore, the sensor 3 according to the invention may optionally be packaged or encapsulated accurately in an encapsulation. Then the sensor 3 may be packaged in a way that wetness, biological analytes and/or chemical analytes found in body fluids, body waste or body exudates to be detected, can penetrate through the package into the sensor 3, e.g. via pores, slots or holes, in the package material.

The sensor 3 may, for example, be utilized to detect an event, such as urination or defecation, after absorption onto or into the absorbent article 2. When the sensor 3 detects an event, a detectable response is generated, which may be detected by the reading means 5. Thereby the sensor 3 enables the user, parent, care taker, nursing personnel, etc. to determine with ease when an event has occurred or when the absorbent article 2 has to be exchanged.

As mentioned above, the machine readable sensor 3 positioned in the absorbent article 2 is a sensor that is realized as a wireless communication means and may, for example, comprise means for communicating a measurement result using radio frequency communication. Thus, the sensor 3 may, for example, comprise an RF tag or may be realized as BlueTooth^{®} or wireless local area network (WLAN) communication means, or radio frequency identification (RFID) communication means.

RFID is a technology in which the electromagnetic or electrostatic coupling in the RF portion of the electromagnetic spectrum is used to transmit signals. An RFID tag comprises typically a device, e.g. a microchip, that stores information. For example, the microchip may include a unique serial number, but it may also include other information. A reader, or interrogator, communicates with the RFID tag through radio waves. The microchip is attached to an antenna that receives signals from and sends signals to the interrogator. Furthermore, RFID tags can be active tags, passive tags or semi-passive tags. Active tags include a power source that powers the microchip's circuitry and transmits a signal to the interrogator. Passive tags do not include a power source. Passive tags draw the power required for the circuitry and the transmission of information from the electromagnetic field generated by the interrogator. Semi-passive tags are similar to active tags; however the power source is used to run the microchip's circuitry, but not to communicate with the interrogator.

For example, the sensor 3 in the system 1 according to the invention may be an RFID tag. The RFID tag may then be any one of the known RFID tags that may be utilized for detection of fluids, e.g. body fluids, body waste or body exudates. For example, the RFID tag may be any one of the RFID tags of the type known in the art of electronic article surveillance (EAS) that operate in the energy absorption mode or in the energy radiation mode. The use of RFID tags of the type known in the art of EAS that operate in the energy absorption mode or in the energy radiation mode as sensors for detection of discharged fluids in absorbent articles is, for example, described in US 6,774,800.

In other embodiments of the system 1 according to the invention the sensor 3 is an RF tag including an inductor-capacitor resonator. The RF tag including an inductor-capacitor may then be any one of the known such tags that may be utilized for detection of fluids, e.g. body fluids, body waste or body exudates. For example, one RF tag including an inductor-capacitor resonator that may be utilized as a sensor for detection of discharged fluids in an absorbent article is described in US 6,774,800.

In still other embodiments of the system 1 according to the invention the sensor 3 is a magnetoelastic sensor. Then the sensor 3 may be any one of the known magnetoelastic sensors that may be utilized for detection of wetness, i.e. liquid, humidity or moisture, a biological analyte and/or a chemical analyte in an absorbent article 2. For example, the sensor 3 may consist of the magnetoelastic sensor described in WO 2004/021944.

If the sensor 3 is a magnetoelastic sensor, it comprises a film of a magnetoelastic material. Suitable materials to be utilized as the magnetoelastic material may be any material with a non-zero magnetostriction and a high magnetoelastic coupling, such as iron-nickel alloys, rare earth metals, ferrites, e.g. spinel type ferrites (Fe₃O₄, MnFe₂O₄), silicon-iron alloys, many other different alloys and mixtures thereof. Soft magnetoelastic materials, alloys and mixtures thereof as well as amorphous magnetoelastic materials, alloys and mixtures thereof may be utilized. Examples of amorphous magnetoelastic alloys are met-glases such as Fe₄₀Ni₃₈Mo₄B₁₈, e.g. Metglas 2826MB^{™} (Honeywell Amorphous Metals, Pittsburg, PA, USA), (FeCo)₈₀B₂₀, (CoNi)₈₀B₂₀ and (FeNi)₈₀B₂₀.

The term "magnetostriction" refers to a phenomenon whereby a material will change dimensions in the presence of an external magnetic field. The size of the dimensional change depends on magnetization in the material and, of course, on the material properties. The phenomenon of magnetostriction is due to the interaction between the atomic magnetic moments in the material.

The term "a high magnetoelastic coupling" refers to the fact that a material having a high magnetoelastic coupling efficiently converts magnetic energy into mechanical elastic energy and vice versa. When a material that may convert magnetic energy into mechanical elastic energy is excited by a time varying magnetic held, elastic waves mechanically deform the material, which has a mechanical resonant frequency inversely proportional to its length. If the material also is magnetostrictive, it generates a magnetic flux when the material is mechanically deformed, which magnetic flux extends remotely and that may be detected by a pick-up coil.

Furthermore, when excited by an external magnetic field, a magnetoelastic material stores magnetic energy in a magnetoelastic mode. When the magnetic field is switched off, the magnetoelastic material shows damped oscillation with a specific frequency denoted as the magnetoacoustic resonant frequency. These oscillations give rise to a magnetic flux that varies in time, which can be remotely detected by a pick-up coil. If a pulsed magnetic field such as, for example, a pulsed sine wave magnetic field is applied to the magnetoelastic material, it will be possible to detect the magnetoacoustic resonant frequency between the pulses. Changes in the magnetoacoustic resonant frequency may be monitored so as to measure or detect multiple environmental parameters.

One way of further enhancing the magnetostrictive effect of the magnetoelastic material in a magnetoelastic sensor is to include a magnetic bias field. For example, a magnetic bias field may be generated by a permanent magnetic film or a permanent magnet positioned in proximity to the magnetoelastic sensor.

If the sensor 3 is a magnetoelastic sensor it may be a wetness sensor, i.e. a sensor detecting wetness such as liquid, humidity or moisture. For example, the film of a magnetoelastic material may then be coated with a wetness sensitive polymer selected from the group consisting of linear and hydrophilic polymers or chemically/physically cross-linked swellable polymer gels based on polyvinyl alcohol, polyvinyl pyrrolidone, polyethylene oxide and co-polymers thereof, polyurethane, polyamides, starch and derivatives thereof, cellulose and derivative thereof, polysaccharides, proteins, polyacrylonitrile, acrylate based polymers, and mixtures thereof. The wetness sensitive polymer may interact with wetness and in such cases change the mass of the sensor 3. For example, the wetness sensitive polymer may interact with wetness through absorption or adsorption. The change in mass of the sensor 3 due to the interaction with wetness will either increase or decrease the resonant frequency, e.g. the magnetoacoustic resonant frequency. The change of the magnetoacoustic resonant frequency is detectable, whereby wetness is delectable. If the sensor 3 is a magnetoelastic sensor detecting wetness it may be utilized for detecting body discharges such as body fluids, body waste or body exudates, i.e. urine, faeces, blood, menstruation blood, fluid matters from wounds and sores, rinsing fluid and saliva.

If the sensor 3 is a magnetoelastic sensor it may alternatively be a sensor adapted to detect at least one biological and/or chemical analyte. Then the film of a magnetoelastic material of the sensor 3 may, instead of being coated with a wetness sensitive polymer, be coated directly or indirectly, i.e. with other layers such as suitable coupling layers in-between, with at least one detector molecule adapted to detect at least one target biological and/or chemical analyte.

The detector molecule may in some embodiments be adapted to detect a biological or chemical analyte selected from the group consisting of an enzyme or a sequence of enzymes; an antibody; a nucleic acid, such as DNA or RNA; a protein, such as a soluble protein or a membrane protein; a peptide, such as an oligopeptide or a polypeptide; an organelle; parts of a natural or synthetic cell membrane or capside, such as a bacterial or a mammalian cell membrane, or a virus capside; an intact or partial viable or nonviable bacterial, plant or animal cell; a piece of plant or mammalian tissues or any other biologically derived molecule; a lipid, a carbohydrate; a lectin, and mixtures thereof.

In other embodiments the detector molecule may be adapted to detect a biological or chemical analyte selected from the group consisting of pathogenic bacteria; non-pathogenic bacteria, e.g. colonic bacteria; viruses; parasites; bacterial toxins; fungi; enzymes; proteins; peptides; mammalian blood cells, such as human white or red blood cells; hormones; mammalian, including human, blood components, such as blood glucose; urine and its components such as glucose, ketones, urobilinogen, and bilirubin; and mixtures thereof.

The bacteria that the detector molecule may be adapted to detect, pathogenic or not, is selected from the group consisting of Escherichia coli, Salmonela typhi, Salmonella paratyphi, Salmonella enteriditid, Salmonella thyphimurium, Salmonella heidelberg, Staphylococcus aureus, Shigella sonnei, Shigella flexneri, Shigella boydii, Shigella dysenteriae, Vbrio cholerae, Mycobacterium tuberculosis, Yersina enterocolitica, Aeromonas hydrophila, Plesmonas shigelloides, Campylobacter jejuni, Campylobacter coli, Bacteroides fragilis, Clostridia septicum, Clostridia perfringens, Clostridia botulinum, Clostridia difficile, and mixtures thereof.

In still other embodiments the detector molecule is adapted to detect a chemical compound or chemical analyte such as health markers or nutritional markers. Nutritional markers include markers for e.g. metabolic efficiency, nutrient deficiencies, nutrient absorption or malabsorption, food and drink intake, food allergies (e.g. to peanuts), food intolerance (e.g. lactose or gluten intolerance), colonic bacteria ecology (e.g. beneficial bacterias such as bifidobacteria and lactobacillus), and total energy balance. Health markers may include chemical analytes such as heavy metals (e.g. lead, mercury, etc.), radioactive substances (e.g. caesium, strontium, uranium, etc.), fats, enzymes, endogenous secretions, protein matter (e.g. blood casts), mucous, and micro-organisms, as described above, that may be related to various health issues such as infection, diarrhoea, gastrointestinal distress of disease, or poisoning. Heavy metals, especially in certain developing countries and in older and/or less affluent areas of developed countries, are serious health risks. For example, lead and mercury poisoning may occur upon the ingestion of these heavy metals from environmental sources (e.g. from lead paint, unregulated heavy industries, etc.) and can be fatal. More commonly, low-level poisoning by these and other heavy metals results in retarded intellectual and/or physical development, especially in children that may occur over a long time and have lasting effects on the individual. Other examples of nutritional markers include calcium, vitamins (e.g. thiamine, riboflavin, niacin, biotin, folic acid, pantothenic acid, absorbic acid, vitamin E, etc.), electrolytes (e.g. sodium, potassium, chlorine, bicarbonate, etc.), fats, fatty acids (long and short chain), soaps (e.g. calcium palmitate), amino acids, enzymes (e.g. lactose, amylase, lipase, trypsin, etc.), bile acids and salts thereof, steroids, and carbohydrates. For example, calcium malabsorption is important in that it may lead to a long-term bone-mass deficiency.

Suitable detector molecules may include any biorecognition element and are further exemplified by carbohydrates, antibodies or parts thereof, synthetic antibodies or parts thereof, enzymes, lectins, DNA (deoxyribonucleic acid), RNA (ribonucleic acid), cells and/or cell membranes or any other molecule with a binding capacity for a defined bio-analyte or chemical analyte.

The detector molecule may, for example, be wholly or partially physiosorbed onto the film of a magnetoelastic material or chemically bound directly or indirectly to the film of a magnetoelastic material.

When a detector molecule coated directly or indirectly on a film of a magnetoelastic material of a magnetoelastic sensor detects a biological or chemical analyte, the mass of the sensor changes resulting in a change of the magnetoacoustic resonant frequency of the sensor. The change of the magnetoacoustic resonant frequency is detectable, whereby the biological or chemical analyte is detectable.

In the system 1 according to the invention, the machine readable identity tag 4 for user identity determination, i.e. for determination of the identity of the user 6 of the absorbent article 2, is arranged to be uniquely associated with the user 6. More specifically, the machine readable identity tag 4 comprises a unique identity, i.e. unique information or data, which is arranged to be uniquely associated with the user 6 of the absorbent article 2. The unique identity may, for example, comprise a unique serial number or a unique resonant frequency. Furthermore, the machine readable identity tag 4 is arranged to be uniquely associated with the user 6 independently of the absorbent article 2, i.e. to be uniquely associated with the user 6 separately from the absorbent article 2, and is not associated with the user 6 via the absorbent article 2. Thereby, the machine readable identity tag 4 is arranged to provide a unique identification to the user 6. The machine readable identity tag 4 is arranged to be positioned at any suitable position on the user 6 or at any suitable position that may be uniquely associated with the user 6, e.g. on the user's bed, on the user's journal or at any other position which may be uniquely associated with the user 6 independently of the absorbent article 2. Thus, the identity tag 4 may, for example, be arranged to be attached to the user 6, whereby the identity tag 4 is arranged to be attached to the user 6 independently of the absorbent article 2, i.e. separately from the absorbent article 2, and it is not included in the absorbent article 2. Furthermore, the machine readable identity tag 4 may optionally be comprised in an attachment means 7 arranged for attachment of the identity tag 4 to the user 6. The attachment means 7 may, for example, be a bracelet, a rubber band, a hook and loop configuration, a belt, a necklace or a shirt. In figure 1, the machine readable identity tag 4 is comprised in a bracelet 7 and is attached to a wrist of the user 6.

Furthermore, the machine readable identity tag 4 is realized as a wireless communication means. For example, the identity tag 4 may be arranged to communicate the unique identity that it comprises using radio frequency communication and may, for example, comprise an RF tag or may be realized as BlueTooth^{®} or wireless local area network (WLAN) communication means, or radio-frequency identification (RFID) communication means.

The machine readable identity tag 4 may, for example, be an RFID tag. The RFID tag may then be any one of the known RFID tags that may be utilized for user identity determination.

Alternatively, instead of being arranged to communicate the unique identity that it comprises using radio frequency communication, the identity tag 4 may comprise an optically readable identity code. The optically readable identity code may, for example, be a bar code. The bar code may then be any one of the known bar codes that may be utilized for user identity determination.

Furthermore, the reading means 5 in the system 1 according to the invention is arranged for performing a reading of the machine readable sensor 3 during the same reading session as a reading of the machine readable identity tag 4. Thereby the reading means 5 is arranged to associate the reading of the sensor 3 with the reading of the identity tag 4. The term "during the same reading session" is herein intended to mean that a reading of the sensor 3 and a reading of the identity tag 4 are performed simultaneously, essentially simultaneously or in sequence. If the two readings are performed in sequence, the sensor reading may be performed before the identity tag reading or vice versa. Furthermore, if the two readings are performed in sequence, the second reading is performed immediately after the first reading or at least within seconds of the first reading. Alternatively, the reading means 5 in the system 1 according to the invention may be arranged for performing a reading of the sensor 3 and a reading of the identity tag 4 during a simultaneous reading session. The term "during a simultaneous reading session" is herein intended to mean that a reading of the sensor 3 and a reading of the identity tag 4 are performed simultaneously or essentially simultaneously.

The reading means 5 in the system 1 according to the invention comprises means for performing a reading of both the machine readable sensor 3 and the machine readable identity tag 4. In an embodiment of the system 1 in which the sensor 3 and the identity tag 4 are based on the different techniques, the reading means 5 comprises two different readers. For example, if the sensor 3 is a magnetoelastic sensor and the identity tag 4 is an RFID tag, the reading means 5 comprises one reader that is able to read the magnetoelastic sensor and one reader that is able to read the RFID tag. However, in an embodiment of the system 1 in which the sensor 3 and the identity tag 4 are based on the same technique, the reading means 5 may comprise two different readers or one reader that may read both of them. For example, if the sensor 3 is an RFID tag and the identity tag 4 also is an RFID tag, the reading means 5 may comprise two different readers or one reader for reading both the sensor 3 and the identity tag 4. A person skilled in the art will realize which reader to use for a certain type of sensor 3 and for a certain type of identity tag 4, respectively. Reference is made to WO 2004/021944 for further details regarding one example of a reader of a magnetoelastic sensor, the excitation of a magnetoelastic material and detection of changes of the magnetoacoustic resonant frequency.

Furthermore, if the reading means 5 comprises two readers, the readers are calibrated to function within essentially the same distance, i.e. the sensor reader is calibrated to perform a reading of the sensor 3 within essentially the same distance as the identity tag reader is calibrated to perform a reading of the identity tag 4. Thereby, the risk is minimized that the reading means 5 performs a reading of a sensor 3 of one user and a reading of an identity tag 4 of another user.

If the reading means 5 comprises two readers, the readers may be positioned in the same unit or in different units. Furthermore, the reading means 5 may optionally further comprise a processing unit for processing the result of the reading of the sensor 3 and the result of the reading of the identity tag 4. If the reading means 5 comprises a processing unit, the processing unit may be a separate unit interconnected with the reader unit(s) or may be a unit integrated with a reader unit. The unit(s) of the reading means 5 is/are preferably hand held. The reading means 5 shown in figure 1 is hand held and comprises one unit.

Furthermore, the reading means 5 comprises means for associating the result of the reading of the sensor 3 with the result of the reading of the identity tag 4 in the same data record. If the reading means 5 comprises a processing unit, the data record may be located in the reading means 5. However, the system 1 may optionally further comprise a central unit 10 such as a personal computer, a lap top computer or a hand held computer and means 9 for communication of the reading means 5 with the central unit 10. The communication means 9 may be wireless or wired. Thus, the reading means 5 may be wirelessly coupled via the communication means 9, e.g. an RF modem, to the central unit 10 or communicatively coupled via the communication means 9, e.g. a computer network, to the central unit 10. The system 1 according to the invention shown in figure 1 comprises a central unit 10 and wireless communication means 9, via which the reading means 5 may communicate with the central unit 10. The data record, in which the result of the reading of the sensor 3 and the result of the reading of the identity tag 4 is associated, may be located in the central unit 10 if a system 1 according to the invention comprises a central unit 10 and communication means 9.

The system 1 according to the invention may comprise any combination of the above described types of absorbent articles 2, the above described types of sensors 3 and the above described types of identity tags 4. Furthermore, for a specific combination of a sensor 3 and an identity tag 4, the system 1 according to the invention may comprise any of the above mentioned configurations of the reading means 5 that is suitable for that specific combination of a sensor 3 and an identity tag 4.

One specific embodiment of the system 1 according to the invention comprises a magnetoelastic sensor as the sensor 3, an RFID tag as the identity tag 4 and a handheld reading means 5 comprising one reader for the magnetoelastic sensor, one reader for the RFID tag and one processing unit. Furthermore, the two readers and the processing unit are in that specific embodiment comprised in the same unit. The magnetoelastic sensor in that specific embodiment may be a wetness sensor or a sensor detecting a biological and/or a chemical analyte. Another specific embodiment of the system 1 according to the invention comprises an RFID tag as the sensor 3, an RFID tag as the identity tag 4 and a handheld reading means 5 comprising a processing unit and one reader that may be utilized to read both RFID tags.

In figure 1 one embodiment of the system 1 according to the invention is shown when applied on a patient 6 by a member 8 of a nursing staff.

Furthermore, the system 1 according to the invention may in other embodiments comprise more than one absorbent article 2 and/or more than one identity tag 4. If the system 1 comprises more than one absorbent article 2 and more than one identity tag 4, it may be utilized for more than one user 6 or patient. If a system 1 comprises more than one absorbent article 2, they may be of the same type or of different types, i.e. the system 1 may then comprise, for example, only diapers or, for example, both diapers and bed protectors.

The present invention provides also a method for associating a reading of a machine readable sensor 3 positioned in an absorbent article 2 with the identity of a user 6 of the absorbent article 2, whereby the absorbent article 2 is arranged to absorb or receive body discharges such as body fluid, body waste or body exudates, i.e. urine, faeces, blood, menstruation blood, fluid matters from wounds and sores, rinsing fluid or saliva, discharged from the user 6, e.g. a patient, an infant, an elderly incontinent person, etc. The absorbent article 2 may be a diaper, a diaper of pant type, an incontinence garment, a sanitary napkin, a tampon-like product, a wound or sore dressing, a bed protector, or a similar product. The sensor 3 may be any of the above described sensors 3 that may be comprised in a system 1 according to the invention.

In the method according to the invention, a machine readable identity tag 4 for determination of the identity of the user 6 of the absorbent article 2 is uniquely associated with the user 6. More specifically, a unique identity, i.e. unique information or data such as a unique serial number or resonant frequency, of the machine readable identity tag 4 is uniquely associated with the user 6. Thereby, the unique identity of the identity tag 4 may, for example, be uniquely associated with the user 6 in a journal, case book, data system or the like. Furthermore, the identity tag 4 is in the method according to the invention uniquely associated with the user 6 independently of the absorbent article 2, i.e. it is uniquely associated with the user 6 separately from the absorbent article 2, and is not associated with the user 6 via the absorbent article 2. Thereby, the identity tag 4 provides a unique identification to the user 6. The identity tag 4 is positioned at any suitable position on the user 6 or at any suitable position which may be uniquely associated with the user 6, e.g. on the user's bed, on the user's journal or at any other position which may be uniquely associated with the user 6 independently of the absorbent article 2. Thus, the identity tag 4 may, for example, be attached to the user 6, whereby the identity tag 4 is attached to the user 6 independently of the absorbent article 2, i.e. separately from the absorbent article 2, and it is not included in the absorbent article 2. The identity tag 4 may be any of the above described identity tags 4 that may be comprised in a system 1 according to the invention and the identity tag 4 may optionally be comprised in any of the above described attachment means 7.

Furthermore, in the method according to the invention the reading of the machine readable sensor 3 positioned in the absorbent article 2 is performed during the same reading session as a reading of the machine readable identity tag 4. Thereby the reading of the sensor 3 and the reading of the identity tag 4 are associated. The term "during the same reading session" is herein intended to mean that a reading of the sensor 3 and a reading of the identity tag 4 are performed simultaneously, essentially simultaneously or in sequence. If the two readings are performed in sequence, the sensor reading may be performed before the identity tag reading or vice versa. Furthermore, if the two readings are performed in sequence, the second reading is performed immediately after the first reading or at least within seconds of the first reading. Alternatively, the reading of the sensor 3 and a reading of the identity tag 4 may be performed during a simultaneous reading session. The term "during a simultaneous reading session" is herein intended to mean that a reading of the sensor 3 and a reading of the identity tag 4 are performed simultaneously or essentially simultaneously.

The reading of the sensor 3 and the reading of the identity tag 4 may be performed by any of the above described reading means 5 that may be comprised in a system 1 according to the invention and that is suitable. The result of the reading of the sensor 3 and the reading of the identity tag 4 may be associated in the same data record. If the reading means 5 comprises a processing unit, the data record may be located in the processing means 5. Otherwise the data record may be located in a central unit 10 to which the reading means 5 is coupled via communication means 9. The communication means 9 may be wireless of wired. Thus, the reading means 5 may be wirelessly coupled via the communication means 9, e.g. an RF modem, to the central unit 10 or communicatively coupled via the communication means 9, e.g. a computer network, to the central unit 10. Furthermore, the result of the reading of the sensor 3 and the reading of the identity tag 4 may be stored either in the processing unit or in the central unit 10 for future comparisons with other results of similar readings for a specific user 6. For example, stored results may be utilized as information of how often a user has been provided with a fresh absorbent article 2 and as information of excretion from the user over time. Furthermore, stored results may be utilized to determine if the type of absorbent article 2 utilized for a specific user 6 should be changed to another type of absorbent article 2 and if different types of absorbent articles 2 for a specific user 6 are needed during the day and during the night.

The risk of associating a reading of a sensor 3 positioned in an absorbent article 2 with an incorrect user identity is reduced by the system 1 and the method according to the invention. This is due to that an identity tag 4 is uniquely associated with the user 6 independently of the absorbent article 2 and that readings of the sensor 3 and the identity tag 4, respectively, are performed during the same reading session, whereby the two readings are automatically associated.

When utilizing the system 1 and method according to the invention, it is not necessary to first perform a reading of a sensor in an absorbent article and after finished reading associate the reading of the sensor with the identity of the user of the absorbent article. Furthermore, when utilizing the system 1 and the method according to the invention, a care taker, a parent, nursing personnel, etc. do not need to move any sensory or reading equipment on the user 6 when changing the absorbent article 2 after it has been soiled. Specifically, since the identity tag 4 is positioned at any suitable position on the user 6 or at any suitable position that may be uniquely associated with the user 6 independently of the absorbent article 2, a care taker, etc. do not need to move the identity tag 4 when changing the absorbent article 2 after it has been soiled. The fact that there is no need to exchange the identity tag 4 each time the absorbent article 2 has to be exchanged implies that a more cost-efficient system is achieved compared to a system in which the identity tag 4 has to be exchanged each time the absorbent article 2 has to be exchanged. Furthermore, the fact that the identity tag 4 does not need to be exchanged each time the absorbent article 2 has to be exchanged, improves the possibility to use a more technically advanced and more expensive identity tag 4.

Furthermore, if the method according to the invention is utilized in a location, such as a daycare centre or a nursing home, where several users 6, or patients, utilize absorbent articles 2 in which a sensor 3 is positioned, it may be automatically determined by the method according to the invention which user 6 is being read.

Even if the absorbent articles 2 described above only comprise one sensor 3, absorbent articles 2, which may be comprised in the system 1 according to the invention and to which the method according to the invention may be applied, may comprise more than one sensor 3. Each sensor 3 in an absorbent article 2 is then arranged to produce a response when read by a reader that is distinguishable from the response of the other sensor(s) 3. Readings of the different sensors 3 in an absorbent article 2 may then be distinguished from each other.

Furthermore, even if the method according to the invention has been described for associating a reading of a machine readable sensor positioned in an absorbent article with the identity of the user of the absorbent article, the method could also be applied for associating a reading of a machine readable sensor positioned in a personal item, such as an article of clothing, with the identity of the person wearing the personal item.

## Claims

1. A method for associating a reading of a machine readable sensor (3) positioned in an absorbent article (2), such as a diaper, a diaper of pant type, an incontinence garment, a sanitary napkin, a tampon-like product, a wound or sore dressing, a bed protector, or a similar product, with the identity of a user (6) of said absorbent article (2), whereby said absorbent article (2) is arranged to absorb or receive body discharges from said user (6),
**characterized in, that**
a machine readable identity tag (4) for determination of the identity of said user (6) is uniquely associated with said user (6) independently of said absorbent article (2), and that
said reading of said machine readable sensor (3) is performed during the same reading session as a reading of the machine readable identity tag (4).

2. The method according to claim 1,
**characterized in, that**
said machine readable identity tag (4) is attached to said user (6) independently of said absorbent article (2).

3. The method according to claim 1 or 2,
**characterized in, that**
said machine readable identity tag (4) is arranged to communicate an identity using radio frequency communication.

4. The method according to claim 3,
**characterized in, that**
said machine readable identity tag (4) is an RFID tag.

5. The method according to claim 1 or 2,
**characterized in, that**
said machine readable identity tag (4) comprises an optically readable identity code.

6. The method according to claim 5,
**characterized in, that**
said optically readable identity code is a bar code.

7. The method according to any of the preceding claims,
**characterized in, that**
said machine readable sensor (3) comprises means for communicating a measurement result using radio frequency communication.

8. The method according to claim 7,
**characterized in, that**
said machine readable sensor (3) is an RFID tag.

9. The method according to any of the preceding claims,
**characterized in, that**
said machine readable sensor (3) is a wetness sensor.

10. The method according to any of claims 1-6,
**characterized in, that**
said machine readable sensor (3) comprises a film of a magnetoelastic material.

11. The method according to claim 10,
**characterized in, that**
said machine readable sensor (3) is a wetness sensor, whereby said film of a magnetoelastic material is coated with a wetness sensitive polymer selected from the group consisting of linear and hydrophilic polymers or chemically/physically cross-linked swellable polymer gels based on polyvinyl alcohol, polyvinyl pyrrolidone, polyethylene oxide and co-polymers thereof, polyurethane, polyamides, starch and derivatives thereof, cellulose and derivative thereof, polysaccharides, proteins, polyacrylonitrile, acrylate based polymers, and mixtures thereof.

12. The method according to claim 10,
**characterized in, that**
said film of a magnetoelastic material is coated directly or indirectly with at least one detector molecule adapted to detect at least one target biological and/or chemical analyte.

13. A system (1) comprising an absorbent article (2), such as a diaper, a diaper of pant type, an incontinence garment, a sanitary napkin, a tampon-like product, a wound or sore dressing, a bed protector, or a similar product, and a machine readable identity tag (4) for determination of the identity of a user (6) of said absorbent article (2), whereby said absorbent article (2) is arranged to be positioned to absorb or receive body discharges from said user (6) and whereby a machine readable sensor (3) is positioned in said absorbent article (2),
**characterized in, that**
said machine readable identity tag (4) is arranged to be uniquely associated with said user (6) independently of said absorbent article (2), and that the system (1) further comprises reading means (5) for performing a reading of said machine readable sensor (3) during the same reading session as a reading of said machine readable identity tag (4), whereby the reading of said machine readable sensor (3) is associated with the identity of said user (6).

14. The system (1) according to claim 13,
**characterized in, that**
said machine readable identity tag (4) is arranged to be attached to said user (6) independently of said absorbent article (2).

15. The system (1) according to claim 13 or 14,
**characterized in, that**
said machine readable identity tag (4) is arranged to communicate an identity using radio frequency communication.

16. The system (1) according to claim 15,
**characterized in, that**
said machine readable identity tag (4) is an RFID tag.

17. The system (1) according to claim 13 or 14,
**characterized in, that**
said machine readable identity tag (4) comprises an optically readable identity code.

18. The system (1) according to claim 17,
**characterized in, that**
said optically readable identity code is a bar code.

19. The system (1) according to any of claims 13-18,
**characterized in, that**
said machine readable sensor (3) comprises means for communicating a measurement result using radio frequency communication.

20. The system (1) according to claim 19,
**characterized in, that**
said machine readable sensor (3) is an RFID tag.

21. The system (1) according to any of claims 13-20,
**characterized in, that**
said machine readable sensor (3) is a wetness sensor.

22. The system (1) according to any of claims 13-18,
**characterized in, that**
said machine readable sensor (3) comprises a film of a magnetoelastic material.

23. The system (1) according to claim 22,
**characterized in, that**
said machine readable sensor (3) is a wetness sensor, whereby said film of a magnetoelastic material is coated with a wetness sensitive polymer selected from the group consisting of linear and hydrophilic polymers or chemically/physically cross-linked swellable polymer gels based on polyvinyl alcohol, polyvinyl pyrrolidone, polyethylene oxide and co-polymers thereof, polyurethane, polyamides, starch and derivatives thereof, cellulose and derivative thereof, polysaccharides, proteins, polyacrylonitrile, acrylate based polymers, and mixtures thereof.

24. The system (1) according to claim 22,
**characterized in, that**
said film of a magnetoelastic material is coated directly or indirectly with at least one detector molecule adapted to detect at least one target biological and/or chemical analyte.

25. The system (1) according to any of the preceding claims,
**characterized in, that**
the system (1) further comprises a central unit (10) and means (9) for communicating with the central unit (10).

26. The system (1) according to claim 25, **characterized in, that** said communication means (9) is wireless or wired.

## Patentansprüche

1. Verfahren zum Assoziieren eines Messwerts eines maschinenlesbaren Sensors (3) in einem absorbierenden Artikel (2), wie zum Beispiel einer Windel, einer Hosenwindel, einem Inkontinenzkleidungsstück, einerDamenbinde, einem tampongleichen Produkt, einem Wundverband, einem Verband für eine wunde Stelle, einem Bettschutz oder einem ähnlichen Produkt, mit der Identität eines Benutzers (6) des absorbierenden Artikels (2),wobei der absorbierende Artikel (2) eingerichtet ist, um Körperausflüsse des Benutzers (6) zu absorbieren oder aufzunehmen,
**dadurch gekennzeichnet, dass**
ein maschinenlesbares Identitätstag (4) zum Bestimmen der Identität des Benutzers (6) unabhängig von dem absorbierenden Artikel (2) eindeutig mit dem Benutzer (6) assoziiert ist und, dass
der Messwert des maschinenlesbaren Sensors (3) während desselben Auslesevorgangs durchgeführt wird, wie das Auslesen des maschinenlesbaren Identitätstags (4).

2. Verfahren gemäß Anspruch 1,
**dadurch gekennzeichnet, dass**
der maschinenlesbare Identitätstag (4) unabhängig von dem absorbierenden Artikel (2) an dem Benutzer (6) angebracht wird.

3. Verfahren gemäß Anspruch 1 oder 2,
**dadurch gekennzeichnet, dass**
der maschinenlesbare Identitätstag (4) eingerichtet wird, um eine Identität unter Verwendung von Funkübertragung zu übertragen.

4. Verfahren gemäß Anspruch 3,
**dadurch gekennzeichnet, dass**
der maschinenlesbare Identitätstag (4) ein RFID-Tag ist.

5. Verfahren gemäß Anspruch 1 oder 2,
**dadurch gekennzeichnet, dass**
der maschinenlesbare Identitätstag (4) einen optisch lesbaren Identitätscode aufweist.

6. Verfahren gemäß Anspruch 5,
**dadurch gekennzeichnet, dass**
der optisch lesbare Identitätscode ein Strichcode ist.

7. Verfahren gemäß einem der vorstehenden Ansprüche,
**dadurch gekennzeichnet, dass**
der maschinenlesbare Sensor (3) ein Mittel zum Übertragen eines Messergebnisses unter Verwendung von Funkübertragung aufweist.

8. Verfahren gemäß Anspruch 7,
**dadurch gekennzeichnet, dass**
der maschinenlesbare Sensor (3) ein RFID-Tag ist.

9. Verfahren gemäß einem der vorstehenden Ansprüche,
**dadurch gekennzeichnet, dass**
der maschinenlesbare Sensor (3) ein Feuchtigkeitssensor ist.

10. Verfahren gemäß einem der Ansprüche 1-6,
**dadurch gekennzeichnet, dass**
der maschinenlesbare Sensor (3) eine Schicht eines magnetoelastischen Materials aufweist.

11. Verfahren gemäß Anspruch 10,
**dadurch gekennzeichnet, dass**
der maschinenlesbare Sensor (3) ein Feuchtigkeitssensor ist, wobei die Schicht eines magnetoelastischen Materials mit einem feuchtigkeitsempfindlichen Polymer beschichtet ist, das aus der Gruppe ausgewählt ist, die lineare und hydrophile Polymereoder chemisch/physikalisch quervernetzte quellfähige Polymergele, basierend auf polyvinylalkohol, Polyvinylpyrrolidon, Polyethylenglycol und Copolymere davon, Polyurethan, Polyamide, Stärke und Derivate davon, Zellulose and Derivate davon, Polysaccharide, Proteine, Polyacrylnitril, acrylsäureesterbasierte Polymere, und Mischungen davon, umfasst.

12. Verfahren gemäß Anspruch 10,
**dadurch gekennzeichnet, dass**
die Schicht eines magnetoelastischen Materials direkt oder indirekt mit mindestens einem Detektionsmolekül beschichtet wird, das eingerichtet ist, um mindestens einen biologischen und/oder chemischen Zielanalyt zu detektieren.

13. System (1) mit einem absorbierenden Artikel (2), wie zum Beispiel einer Windel, einer Windelhose, einem Inkontinenzkleidungsstück, einer Danenbinde, einem tampongleichen Produkt, einem Wundverband, einem Verband für eine wunde Stelle, einem Bettschutz oder einem ähnlichen Produkt und einem maschinenlesbaren Identitätstag (4) zur Bestimmung der Identität eines Benutzers (6) des absorbierenden Artikels (2), wobei der absorbierende Artikel (2) eingerichtet ist, um zum Absorbieren oder Aufnehmen von Körperausflüssen des Benutzers (6) angeordnet zu sein und wobei ein maschinenlesbarer Sensor (3) in dem absorbierenden Artikel (2) angeordnet ist,
**dadurch gekennzeichnet, dass** der maschinenlesbare Identitätstag (4) eingerichtet ist, um unabhängig von dem absorbierenden Artikel (2) eindeutig mit dem Benutzer (6) assoziiert zu sein und dadurch, dass das System (1) des Weiteren Auslesemittel (5) zum Auslesen des maschinenlesbaren Sensors (3) während desselben Auslesevorgangs wie ein Auslesen des maschinenlesbaren Identitätstags (4) aufweist, wobei das Auslesen des maschinenlesbaren Sensors (3) mit der Identität des Benutzers (6) assoziiert ist.

14. System (1) gemäß Anspruch 13,
**dadurch gekennzeichnet, dass**
der maschinenlesbare Identitätstag (4) eingerichtet: ist, um unabhängig von dem absorbierenden Artikel (2) an dem Benutzer (6) angebracht zu sein.

15. System (1) gemäß Anspruch 13 oder 14,
dadurch gekenntzeichnet, dass
der maschinenlesbare Identitätstag (4) eingerichtet ist, um eine Identität unter Verwendung von Funkübertragung zu übertragen.

16. System (1) gemäß Anspruch 15,
**dadurch gekennzeichnet, dass**
der maschinenlesbare Identitätstag (4) ein RFID-Tag ist.

17. System (1) gemäß Anspruch 13 oder 14,
**dadurch gekennzeichnet, dass**
der maschinenlesbare Identitätstag (4) einen optisch lesbaren Identitätscode aufvreist.

18. System (1) gemäß Anspruch 17,
dadurch gekenntzeichnet, dass
der optisch lesbare Identitätscode ein Strichcode ist.

19. System (1) gemäß einem der Ansprüche 13-18,
**dadurch gekennzeichnet, dass**
der maschinenlesbare Sensor (3) ein Mittel zum Übertragen eines Messeergebnisses unter Verwendung von Funkübertragungaufweist.

20. System (1) gemäß Anspruch 19,
**dadurch gekennzeichnet, dass**
der maschinenlesbare Sensor (3) einer RFID-Tag ist.

21. System (1) gemäß einem der Ansprüche 13-20,
**dadurch gekennzeichnet, dass**
der maschinenlesbare Sensor (3) ein Feuchtigkeitssensor ist.

22. System (1) gemäß einem der Ansprüche 13-18,
**dadurch gekennzeichnet, dass**
der maschinenlesbare Sensor (3) eine Schicht eines magnetoelastischen Materials aufweist.

23. System (1) gemäß Anspruch 22,
**dadurch gekennzeichnet, dass**
der maschinenlesbare Sensor (3) ein Feuchtigkeitssensor ist, wobei die Schicht eines magnetoelastischen Materials mit einem feuchtigkeitsempfindlichen Polymer beschichtet ist, das aus der Gruppe ausgewählt ist, die lineare und hydrophile Polymereoder chemisch/physikalisch quervernetzte quellfähige Polymergele, basierend auf Polyvinylalkohol, Polyvinylpyrrolidon, Polyethylenglycol und Copolymere davon, Polyurethan, Polyamide, Stärke und Derivate davon, Zellulose und Derivate davon, Polysaccharide, Proteine, Polyacrylnitril, acrylsäureesterbasierte Polymere, und Mischungen davon, umfasst.

24. System (1) gemäß Anspruch 22,
**dadurch gekennzeichnet, dass**
die Schicht eines magnetoelastischen Materials direkt oder indirekt mit mindestens einem Detektionsmolekül beschichtet wird, das eingerichtet ist, um mindestens einen biologischen und/oder chemischen Zielanalyt zu detektieren.

25. System (1) gemäß einem der vorstehenden Ansprüche,
**dadurch gekennzeichnet, dass**
das System (1) des Weiteren eine zentrale Einheit (10) und ein Mittel (9) zum Kommunizieren mit der zentralen Einheit (10) aufweist.

26. System (1) gemäß Anspruch 25,
**dadurch gekennzeichnet, dass**
das Übertragungamittel (9) drahtlos oder drahtgebunden ist.

## Revendications

1. Procédé pour associer une lecture d'un capteur (3) lisible par une machine, positionné dans un article absorbant (2) comme une couche, une couche-culotte, une protection contre l'incontinence, une serviette hygiénique, un article de type tampon, un pansement, un protège matelas ou article similaire, avec l'identité d'un utilisateur (6) dudit article absorbant (2), ledit article absorbant (2) étant agencé pour absorber ou recevoir des écoulements corporels provenant dudit utilisateur (6),
**caractérisé en ce que**
une étiquette d'identité (4) lisible par une machine pour permettre la détermination de l'identité dudit utilisateur (6) est associée de façon unique audit utilisateur (6) indépendamment dudit article absorbant (2), et **en ce que**
ladite lecture du capteur (3) lisible par une machine est effectuée pendant la même session de lecture qu'une lecture de l'étiquette d'identité (4) lisible par une machine.

2. Procédé selon la revendication 1, **caractérisé en ce que** ladite étiquette d'identité (4) lisible par une machine est attachée audit utilisateur (6) indépendamment dudit article absorbant (2).

3. Procédé selon la revendication 1 ou 2, **caractérisé en ce que** ladite étiquette d'identité (4) lisible par une machine est adaptée pour communiquer une identité en utilisant une communication par radiofréquence.

4. Procédé selon la revendication 3, **caractérisé en ce que** ladite étiquette d'identité (4) lisible par une machine est une étiquette RFID.

5. Procédé selon la revendication 1 ou 2, **caractérisé en ce que** ladite étiquette d'identité (4) lisible par une machine comprend un code d'identité lisible de façon optique.

6. Procédé selon la revendication 5, **caractérisé en ce que** ledit code d'identité lisible de façon optique est un code à barres.

7. Procédé selon l'une quelconque des revendications précédentes, **caractérisé en ce que** ledit capteur (3) lisible par une machine comprend un moyen permettant de communiquer un résultat de mesure en utilisant une communication par radiofréquence.

8. Procédé selon la revendication 7, **caractérisé en ce que** ledit capteur (3) lisible par une machine est une étiquette RFID.

9. Procédé selon l'une quelconque des revendications précédentes, **caractérisé en ce que** ledit capteur (3) lisible par une machine est un capteur d'humidité.

10. Procédé selon l'une quelconque des revendications 1 à 6, **caractérisé en ce que** ledit capteur (3) lisible par une machine comprend un film fait d'un matériau magnétostrictif.

11. Procédé selon la revendication 10, **caractérisé en ce que** ledit capteur (3) lisible par une machine est un capteur d'humidité, ledit film fait d'un matériau magnétostrictif étant revêtu d'un polymère sensible à l'humidité choisi dans le groupe comprenant les polymères linéaires et hydrophiles ou les gels de polymère capables de gonfler réticulés chimiquement ou physiquement à base d'alcool polyvinylique, de polyvinylpyrrolidone, d'oxyde de polyéthylène et de ses copolymères, de polyuréthane, de polyamides, d'amidon et de ses dérivés, de cellulose et de ses dérivés, de polysaccharides, de protéines, de polyacrylonitrile, de polymères à base d'acrylate, et leurs mélanges.

12. Procédé selon la revendication 10, **caractérisé en ce que** ledit film fait d'un matériau magnétostrictif est revêtu directement ou indirectement d'au moins une molécule détectrice adaptée pour détecter au moins un analyte biologique et/ou chimique ciblé.

13. Système (1) comprenant un article absorbant (2) comme une couche, une couche-culotte, une protection contre l'incontinence, une serviette hygiénique, un article de type tampon, un pansement, un protège matelas ou article similaire, et une étiquette d'identité (4) lisible par une machine pour permettre la détermination de l'identité d'un utilisateur (6) dudit article absorbant (2), ledit article absorbant (2) étant adapté pour être positionné pour absorber ou recevoir des écoulements corporels provenant dudit utilisateur (6), et un capteur (3) lisible par une machine étant positionné dans ledit article absorbant (2),
**caractérisé en ce que** ladite étiquette d'identité (4) lisible par une machine est adaptée pour être associée de façon unique audit utilisateur (6) indépendamment dudit article absorbant (2), et **en ce que**
le système (1) comprend en outre un moyen de lecture (5) pour effectuer une lecture dudit capteur (3) lisible par une machine pendant la même session de lecture qu'une lecture de ladite étiquette d'identité (4) lisible par une machine, moyennant quoi la lecture dudit capteur (3) lisible par une machine est associée à l'identité dudit utilisateur (6).

14. Système (1) selon la revendication 13, **caractérisé en ce que** ladite étiquette d'identité (4) lisible par une machine est adaptée pour être attachée audit utilisateur (6) indépendamment dudit article absorbant (2).

15. Système (1) selon la revendication 13 ou 14, **caractérisé en ce que** ladite étiquette d'identité (4) lisible par une machine est adaptée pour communiquer une identité en utilisant une communication par radiofréquence.

16. Système (1) selon la revendication 15, **caractérisé en ce que** ladite étiquette d'identité (4) lisible par une machine est une étiquette RFID.

17. Système (1) selon la revendication 13 ou 14, **caractérisé en ce que** ladite étiquette d'identité (4) lisible par une machine comprend un code d'identité lisible de façon optique.

18. Système (1) selon la revendication 17, **caractérisé en ce que** ledit code d'identité lisible de façon optique est un code à barres.

19. Système (1) selon l'une quelconque des revendications 13 à 18, **caractérisé en ce que** ledit capteur (3) lisible par une machine comprend un moyen permettant de communiquer un résultat de mesure en utilisant une communication par radiofréquence.

20. Système (1) selon la revendication 19, **caractérisé en ce que** ledit capteur (3) lisible par une machine est une étiquette RFID.

21. Système (1) selon l'une quelconque des revendications 13 à 20, **caractérisé en ce que** ledit capteur (3) lisible par une machine est un capteur d'humidité.

22. Système (1) selon l'une quelconque des revendications 13 à 18, **caractérisé en ce que** ledit capteur (3) lisible par une machine comprend un film fait d'un matériau magnétostrictif.

23. Système (1) selon la revendication 22, **caractérisé en ce que** ledit capteur (3) lisible par une machine est un capteur d'humidité, ledit film fait d'un matériau magnétostrictif étant revêtu d'un polymère sensible à l'humidité choisi dans le groupe comprenant les polymères linéaires et hydrophiles ou les gels de polymère capables de gonfler réticulés chimiquement ou physiquement à base d'alcool polyvinylique, de polyvinylpyrrolidone, d'oxyde de polyéthylène et de ses copolymères, de polyuréthanne, de polyamides, d'amidon et de ses dérivés, de cellulose et de ses dérivés, de polysaccharides, de protéines, de polyacrylonitrile, de polymères à base d'acrylate, et leurs mélanges.

24. Système (1) selon la revendication 22, **caractérisé en ce que** ledit film fait d'un matériau magnetostrictif est revêtu directement ou indirectement d'au moins une molécule détectrice adaptée pour détecter au moins un analyte biologique et/ou chimique ciblé.

25. Système (1) selon l'une quelconque des revendications précédentes, **caractérisé en ce que** le système (1) comprend en outre une unité centrale (10) et un moyen (9) pour communiquer avec l'unité centrale (10).

26. Système (1) selon la revendication 25, **caractérisé en ce que** ledit moyen de communication (9) est sans fil ou filaire.
